# EUROPEAN PATENT SPECIFICATION

(11) **EP 3 858 783 B1**
(45) Date of publication and mention of the grant of the patent: **16.07.2025**
(21) Application number: 19866519.2
(22) Date of filing: 16.05.2019
(51) Int. Cl.: B67C 7/00, B65B 55/02, A61L 2/00

(54) **STERILIZING PROCESS USED FOR STERILE FILLING PRODUCTION LINE**
STERILISATIONSVERFAHREN FÜR EINE PRODUKTIONSLINIE ZUR STERILEN ABFÜLLUNG
PROCÉDÉ DE STÉRILISATION UTILISÉ POUR UNE LIGNE DE PRODUCTION DE REMPLISSAGE STÉRILE

(30) Priority: 25.09.2018 CN 201811119440
(43) Date of publication of application: 04.08.2021
(73) Proprietor: Jiangsu Newamstar Packaging Machinery Co., Ltd, Suzhou, Jiangsu 215618 (CN)
(72) Inventor: MAO, Jiahui, Suzhou, Jiangsu 215618 (CN); YANG, Yajun, Suzhou, Jiangsu 215618 (CN)
(74) Representative: Murgitroyd & Company
(86) International application number: PCT/CN2019/087199
(87) International publication number: WO 2020/062889

(56) References cited:
- CN-A- 1 837 023
- CN-A- 101 489 910
- CN-A- 101 489 910
- CN-A- 107 670 058
- CN-A- 109 502 532
- JP-A- 2008 074 438
- JP-A- H07 155 150
- JP-A- H07 155 150

## Description

### Technical Field of the Invention

The present invention relates to a sterilizing process used for an aseptic filling production line.

### Background of the Invention

In the aseptic filling production line, the material sterilizer and aseptic water sterilizer are essential supporting equipment, which must be operated simultaneously to provide the corresponding processing medium for the aseptic filling machine.

The material sterilizer is used for the ultra-high temperature instantaneous sterilization of product materials at 138 to 140 °C, which can effectively kill *Escherichia coli*, fungi, pathogenic bacteria, etc. in the materials, and at the same time destroy the microorganisms and spores that can grow in them, so that the final products after filling can achieve the purposes of long-term transportation and storage, and the ultra-high temperature instantaneous sterilization extends the shelf life of beverage products. The aseptic water sterilizer is used in the production of aseptic filling to wash the bottles and caps with aseptic water after being sterilized by the disinfectant, to ensure that the residue in the bottles and caps used for beverage packaging used before the product is filled and capped is washed without any residue left, and the generation of aseptic water is also through the use of ultra-high temperature instantaneous sterilization equipment to thoroughly sterilize the water entering the filling environment at 140 °C.

The medium for 140 °C sterilization by heat exchange in the material sterilizer is high-temperature water, which requires that the heating temperature of the high-temperature section is as high as 143 to 145 °C, that is, higher than the 140 °C sterilization temperature of the ordinary aseptic water sterilizer, which can fully meet the requirements of aseptic water sterilization process. The pipeline in the hot water system of the material sterilizer is a fully enclosed structure with pressure, which fully meets the aseptic process conditions.

In the material sterilizer, the final temperature of the high-temperature water after heat exchange with the materials is between 50 - 60 °C, and the high-temperature water is used to cool the materials after backflow; while in the aseptic water sterilizer, the use temperature of aseptic water is 50 °C. The high-temperature water of 50 - 60 °C has a poor cooling effect on the materials, and when a large amount of pure water is heated to the sterilization temperature and then cooled to the use temperature, it consumes a lot of pure water and energy.

JP H07155150A is a method of sterilizing canned food by means of a sterilization kettle, which can be regarded as useful to understand the invention.

### Summary of the Invention

The invention is defined by the claims.

The present invention is aimed at providing a sterilizing process used for an aseptic filling production line, in which, high-temperature water generated in a high-temperature water circulation system is heated and sterilized to directly convert it into aseptic water for supply and use, and the aseptic water is passed into a sterile water circulation system through a first pipeline after cooling the material. At the same time, the flow of self-supplied aseptic water in the aseptic water circulation system is gradually reduced, and in the aseptic water circulation system, as the production capacity is gradually reduced, the amount of heat exchange steam used for heating and sterilization will also be reduced, effectively reducing the energy consumption index of the steam, ice water and tower water of the aseptic water circulation system during operation, thus greatly reducing the operation input cost, and as long as the aseptic water circulation system can always ensure the normal operation of the small flow rate, and ensure the aseptic environment is not destroyed, the smaller the self-flow rate is reduced, the lower the overall operating energy consumption; at the same time, the high-temperature water circulating in the high-temperature water circulation system will also be reduced, and pure water at room temperature to the high-temperature water circulation system is added to directly recover heat and exchange heat with the high-temperature material in the material circulation system , and the newly added normal-temperature pure water has a lower temperature than the high-temperature water that is normally backflowed, the heat recovery and cooling capacity on the materials will be better, and the temperature of the materials can be lowered lower, and it can effectively reduce the energy consumption of media such as tower water and ice water at the rear end of material cooling.

To achieve the above purpose, the technical solution employed by the present disclosure is:
A sterilizing process used for a sterile filling production line, comprises the following steps:
(1) providing a first pipeline between a high-temperature water circulation system and an aseptic water circulation system, and conveying part of high-temperature water that having exchanged heat with materials is to the aseptic water circulation system through the first pipeline;
(2) supplementing the aseptic water circulation system with pure water, and circulating the pure water in the aseptic water circulation system and heating it to a second sterilization temperature; respectively blocking the communication states between the first pipeline and the high-temperature water circulation system and the aseptic water circulation system, then inletting high-temperature water steam with the second sterilization temperature into the first pipeline, until the high-temperature water steam in the first pipeline is kept at the first temperature for a second time period; supplementing the material circulation system with pure water, and supplementing the high-temperature water circulation system with pure water as well, and circulating the pure water in the material circulation system and the high-temperature water circulation system and heating it to the second sterilization temperature ; pre-cleaning a material circulation system, the high-temperature water circulation system, the aseptic water circulation system and the first pipeline, and after the cleaning is qualified, running the material circulation system, the high-temperature water circulation system and the aseptic water circulation system and communicating the high-temperature water circulation system and the aseptic water circulation system to the first pipeline; when the material circulation system and the aseptic water circulation system are both in the normal production state, the first pipeline is communicated to the high-temperature water circulation system and the aseptic water circulation system ; (3) in the high-temperature water circulation system, when the high-temperature water is heated to a first sterilization temperature, sterilizing the high-temperature water for a first time period of heat preservation to obtain aseptic high-temperature water; in the high-temperature water circulation system, before the high-temperature water exchanges heat with steam, it is divided into two paths, the first path directly exchanges heat with the steam, and the second path first exchanges heat with the first path that having exchanged heat with steam, and then flows back into the first path before heat exchange with steam, the first path exchanges heat with the material after exchanging heat with the second path, and a temperature of the first path during heat exchange with the material is a first set temperature; after the first path exchanges heat with the steam, it is heated to the first sterilization temperature, and the temperature is maintained for the first time period; the first set temperature is higher than a temperature of the material exchanging heat with the first path within 3 °C; a material-hot water temperature regulation proportional control valve is provided in the high-temperature water circulation system to regulate the first set temperature;
(4) as the first pipeline gradually supplements the aseptic high-temperature water into the aseptic water circulation system, gradually reducing the flow of the self-supplied aseptic water in the aseptic water circulation system, and at the same time, supplementing the high-temperature water circulation system with room-temperature pure water;
a material-hot water system constant pressure valve and an aseptic hot water supply constant pressure valve are provided in the high-temperature water circulation system, and the material-hot water system constant pressure valveis located upstream of the communication part of the first pipeline and the high-temperature water circulation system, the aseptic hot water supply constant pressure valve is located downstream of the communication part of the first pipeline and the high-temperature water circulation system; an aseptic water cooling system constant pressure valve is provided in the aseptic water circulation system, and the aseptic water cooling system constant pressure valve is located downstream of the communication part of the first pipeline and the aseptic water circulation system; the back pressure of the material-hot water system constant pressure valve is greater than the back pressure of the aseptic hot water supply constant pressure valve which is greater than the back pressure of the aseptic water cooling system constant pressure valve so that the first pipeline can replenish aseptic high-temperature water into the aseptic water circulation system;
the water supply of the aseptic water circulation system is provided by an aseptic water SIP tank or an aseptic water balancing tank, and the aseptic water sequentially passes through a first heat exchanger, a second heat exchanger, a first steam heat exchanger, and then flows back through the second heat exchanger, the first heat exchanger, a third heat exchanger, and an aseptic filling machine, then returns to the aseptic water SIP tank or the aseptic water balancing tank, the first heat exchanger and the second heat exchanger are used to exchange heat between the aseptic water in the shell side and the tube side, and the first steam heat exchanger is used to circulate and heat the aseptic water in the aseptic water circulation system to a second sterilization temperature to obtain the aseptic water, the aseptic water cooled by the third heat exchanger enters the aseptic filling machine for cleaning packaging materials;
the materials of the material circulation system is supplied by a material balancing tank, the materials passes through a fourth heat exchanger to heat up and then passes through a degassing homogenizer, a fifth heat exchanger, and a sixth heat exchanger to heat up and sterilize, the sterilized materials passes through a seventh heat exchanger to heat recovery, then passes through an eighth heat exchanger, and a ninth heat exchanger to cool down, and then goes through the aseptic filling machine for filling;
the hot water of the high-temperature water circulation system is supplied by a hot water tank, the hot water first passes through the seventh heat exchanger to perform a heat recovery with the high-temperature material of the material circulation system, then the hot water is divided into two paths, the first path directly enters a second steam heat exchanger to heat up and sterilize, and then enters a tenth heat exchanger, and the second path enters the tenth heat exchanger to adjust the temperature of the first path, so that the temperature of the high-temperature water output from the tenth heat exchanger and about to enter the sixth heat exchanger is higher than the temperature of the material output from the sixth heat exchanger within 3 °C; the high-temperature water after the heat exchange in the sixth heat exchanger exchanges heat for the low-temperature materials in the material circulation system, and partly returns to the hot water tank, and partly enters the first heat exchanger through the first pipeline, mixes with the aseptic water in the first heat exchanger and exchanges heat, and then passes through the third heat exchanger to cool down, and then supplies together with the aseptic filing machine for use.

Preferably, in the high-temperature water circulation system, the high-temperature water that having exchanged heat with the material enters the aseptic water circulation system through the first pipeline, and then is cooled for use.

Preferably, in step (4), the supplemented room-temperature pure water is mixed with the high-temperature water, and then directly exchanges heat with the material to cool the material.

Due to the use of the above technical solution, the present invention has the following advantages over the prior art: in the sterilizing process used for an aseptic filling production line of the present disclosure, high-temperature water generated in a high-temperature water circulation system is heated and sterilized to directly convert it into aseptic water for supply and use, and the aseptic water is passed into an aseptic water circulation system through a first pipeline after cooling the material. At the same time, the flow of self-supplied aseptic water in the aseptic water circulation system is gradually reduced, and in the aseptic water circulation system, as the production capacity is gradually reduced, the amount of heat exchange steam used for heating and sterilization will also be reduced, effectively reducing the energy consumption index of the steam, ice water and tower water of the aseptic water circulation system during operation, thus greatly reducing the operation input cost, and as long as the aseptic water circulation system can always ensure the normal operation of the small flow rate, and ensure the aseptic environment is not destroyed, the smaller the self-flow rate is reduced, the lower the overall operating energy consumption; at the same time, the high-temperature water circulating in the high-temperature water circulation system will also be reduced, and pure water at room temperature to the high-temperature water circulation system is added to directly recover heat and exchange heat with the high-temperature material in the material circulation system , and the newly added normal-temperature pure water has a lower temperature than the high-temperature water that is normally backflowed, the heat recovery and cooling capacity on the materials will be better, and the temperature of the materials can be lowered lower, and it can effectively reduce the energy consumption of media such as tower water and ice water at the rear end of material cooling.

### Brief Description of the Drawings

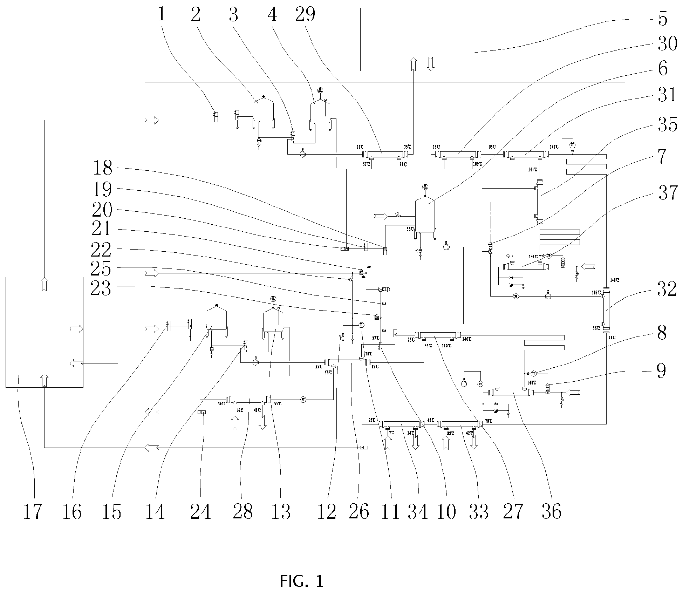

Figure 1 is a schematic structure diagram of an aseptic filling production line applying the process of the present invention.

Wherein, 1 - material backflow tank shut-off valve; 2 - material balancing tank; 3 - material outlet shut-off valve; 4 - material SIP tank; 5 - degassing homogenizer; 6 - hot water tank; 7 - material-hot water temperature regulation proportional control valve; 8 - aseptic water sterilization temperature detector; 9 - aseptic water sterilization temperature proportional valve ;10 - aseptic water sterile T-type diaphragm valve; 11 - shielding temperature detector; 12 - steam SIP discharge valve; 13 - aseptic water SIP tank; 14 - aseptic water outlet shut-off valve; 15 - aseptic water balancing tank; 16 - aseptic water backflow shut-off valve; 17 - aseptic filling machine; 18 - aseptic hot water supply constant pressure valve; 19 - aseptic hot water sterile T-type diaphragm valve; 20 - material-hot water system constant pressure valve; 21 - SIP/shielding steam inlet valve; 22 - steam condensate quick drain valve; 23 - SIP/shielding steam outlet valve; 24 - aseptic water cooling system constant pressure valve; 25 - first pipeline; 26 - first heat exchanger; 27 - second heat exchanger; 28 - third heat exchanger; 29 - fourth heat exchanger; 30 - fifth heat exchanger; 31 - sixth heat exchanger; 32 - seventh heat exchanger; 33 - eighth heat exchanger; 34 - ninth heat exchanger; 35 - tenth heat exchanger; 36 - first steam heat exchanger; 37 - second steam heat exchanger.

### Detailed Description of Exemplary Embodiments

In the following, the technical solution of the present invention is further described combining with the accompanying drawings.

As shown in Figure 1, to combine a material sterilizer and an aseptic water sterilizer into an all-in-one machine, a first pipeline 25 is added between a high-temperature water circulation system and an aseptic water circulation system.

### Before work:

First, SIP(steam-in-place) cleaning is performed on the aseptic water circulation system, the water supply is provided by an aseptic water SIP tank 13, and the aseptic water sequentially passes through a first heat exchanger 26, a second heat exchanger 27, a first steam heat exchanger 36, and then flows back through a second heat exchanger 27, a first heat exchanger 26, a third heat exchanger 28, and an aseptic filling machine 17, then returns to the aseptic water SIP tank 13. The first heat exchanger 26 and the second heat exchanger 27 are used to exchange heat between the aseptic water in the shell side and the tube side, and the first steam heat exchanger 36 is used to circulate and heat the aseptic water in the aseptic water circulation system to a second sterilization temperature, and keep for 30 min. In the current embodiment, the second sterilization temperature is 143 °C.

Then SIP cleaning is performed on the first pipeline 25, the medium is high-temperature steam at 143 °C (the second sterilization temperature), and when the steam SIP starts, a steam condensate quick drain valve 22 and a steam SIP discharge valve 12 are both opened, and the steam condensate is discharged quickly, and after the condensate are discharged, the steam condensate quick drain valve 22 is closed separately, and an SIP/shielded steam inlet valve 21 is opened at the same time, SIP high-temperature pure steam enters the entire first pipeline 25, and the 143 °C high-temperature steam passes through an aseptic hot water supply control valve, then enters the steam SIP discharge valve 12 through an opened SIP/shielding steam outlet valve 23 for rapid discharge, and when a shielding temperature detector 11 detects that the sterilization temperature is 138 °C (a first temperature) which is qualified, the steam SIP discharge valve 12 is closed separately, and heat preservation and sterilization performed on the steam is timed for 30 minutes (a second time period); when the shielding temperature detector 11 detects that the sterilization temperature is lower than the timed sterilization temperature of 138 °C by more than 2 °C, the steam SIP discharge valve 12 is opened again for quick discharge and temperature rise, and it will enter the 30-minute timing state after returning to the temperature; during the steam sterilization process of the entire first pipeline 25, an aseptic water sterile T-type diaphragm valve 10 on the lower end and an aseptic hot water sterile T-type diaphragm valve 19 on the upper end are both closed; and after the steam sterilization of the entire first pipeline 25 finishes, high-temperature pure steam still needs to be passed in, and pure steam is used for shielding protection to ensure that the upper and lower pipelines are always in the aseptic state, at this moment, a valve cavity of the aseptic hot water aseptic T-type diaphragm valve 19 has not yet started SIP sterilization and is in a bacterial state.

Then SIP cleaning is performed on the material circulation system and the high-temperature water circulation system at the same time, water is supplied by a material SIP tank 4 and a hot water tank 6, respectively, and aseptic water in the material SIP tank 4 successively passes through a fourth heat exchanger 29 and a degassing homogenizer 5, a fifth heat exchanger 30, and a sixth heat exchanger 31 to heat up, and then passes through a seventh heat exchanger 32, an eighth heat exchanger 33, and a ninth heat exchanger 34 to cool down, and then goes through the aseptic filling machine 17, returns to the material SIP tank 4; aseptic water in the hot water tank 6 successively passes through the seventh heat exchanger 32 and the second steam heat exchanger 37 to heat up, and then a tenth heat exchanger 35 to adjust to a first set temperature, and passes through the sixth heat exchanger 31, the fifth heat exchanger 30, and the fourth heat exchanger 29 to cool down, and finally returns to the hot water tank 6. The second steam heat exchanger 37 is used to circulate and heat the aseptic water in the material circulation system and the high-temperature water circulation system to 143 °C (the second sterilization temperature), and keep for 30 min.

### When working:

First of all, the aseptic water side is in the normal supply state, and at this time, an aseptic water outlet shut-off valve 14 is closed, the system water supply is supplied by an aseptic water balancing tank 15, and the aseptic water backflow shut-off valve 16 is closed, and the backflow is switched back to the aseptic water balancing tank 15 at the same time, and the entire system generates a loop. The SIP sterilization temperature is set to 140 °C by an aseptic water sterilization temperature detector 8, and the opening of the aseptic water sterilization temperature proportional valve 9 is adjusted to control the heat-exchange steam volume, and to adjust the sterilization temperature, and the aseptic water is sterilized and then cooled to 50 °C to continuously supply to the aseptic filling machine 17; and the entire first pipeline 25 is in the state of shielding above the temperature of 125 °C using pure steam.

Then the material outlet shut-off valve 3 on the material side is closed, the system feed is supplied by the material balancing tank 2, and the material backflow tank shut-off valve 1 is closed at the same time, the backflow is simultaneously switched back to the material balancing tank 2, and the entire system generates a loop. The material sterilization production starts, the material enters the material balancing tank 2 for buffering, and then is heated to 75 °C through the material degassing heat exchanger, and then processed in the degassing homogenizer 5, and then returned to the fifth heat exchanger 30 to continue heating, then enters the sixth heat exchanger 31 to heat up to 140 °C sterilization temperature to keep for 30 s of sterilization, enters the seventh heat exchanger 32 for heat recovery, and then enters the eighth heat exchanger 33 and the ninth heat exchanger 34 to cool down to 25 °C and goes to the aseptic filling machine 17 for filling.

The hot water that exchanging heat with the material is provided by the hot water tank 6, and the hot water first passes through the seventh heat exchanger 32 to perform a heat recovery with the 140 °C high-temperature material, heats up from about 50 °C to above 100 °C, then it is divided into two paths, the first path directly enters the second steam heat exchanger 37 and is heated to 144 °C (the first sterilization temperature) and then enters the tenth heat exchanger 35, and the second path enters the tenth heat exchanger 35 to adjust the temperature of the first path, so that the temperature of the high-temperature water output from the tenth heat exchanger 35 and about to enter the sixth heat exchanger 31 is higher than the temperature of the material output from the sixth heat exchanger 31 within 3 °C. Specifically, the first set temperature is controlled by the material-hot water temperature regulation proportional control valve 7. Through this setting, it is possible to prevent the scale formation of material due to the excessive temperature difference when the temperature is raised. In the current embodiment, the first set temperature is also 143 °C, and in the sixth heat exchanger 31, the outlet temperature of the material is 140 °C. Since the sterilization temperature of acidic materials needs to be adjusted at a sterilization temperature of 125 - 140 °C, and it is necessary to ensure that the high-temperature water can reach 143 °C sterilization process to achieve sterility, and the purpose of sterilizing materials at different temperatures must be met, the material-hot water temperature regulation proportional control valve 7 performs recovery adjustment to control the amount of heat recovery to realize the cooling of the material; the high-temperature water after the heat exchange is then exchanged heat with the low-temperature material at the rear end to be finally cooled to about 50 °C - 60 °C and backflowed to the hot water tank 6. After the heat exchange between the first path and the second steam heat exchanger 37, the temperature is raised to 144 °C, and kept for 30 s to sterilize to obtain aseptic high-temperature water.

When the aforementioned aseptic water side and the material side are both in the normal production state in which the aseptic filling machine 17 is supplied normally, the material aseptic hot water supply can be started.

Both SIP/shielding steam inlet valve 21 and SIP/shielding steam outlet valve 23 are closed to stop the steam shielding of the first pipeline 25, and at the same time, the steam condensate quick drain valve 22 is opened to perform steam shielding protection on an external condensate drain pipeline, and the shielding temperature can be detected by the shielding temperature detector 11 to reach 125 °C, and when the detection value of the shielding temperature detector 11 is lower than the shielding temperature of 125 °C by more than 2 °C, the steam SIP discharge valve 12 opens again for rapid discharge and temperature rise, and when the detection value of the shielding temperature detector 11 returns to over 125 °C, it will be closed to continue the heat preservation to maintain the external shielding state.

At this time, the aseptic water sterile T-type diaphragm valve 10 and the aseptic hot water sterile T-type diaphragm valve 19 at the upper and lower ends can be opened, the back pressure of a material-hot water system constant pressure valve 20 is set to the maximum, and then the back pressure of an aseptic hot water supply constant pressure valve 18 is set to be less than the back pressure of the material-hot water system constant pressure valve 20, but must be greater than the back pressure of an aseptic water cooling system constant pressure valve 24, so that the aseptic high-temperature water at the high pressure end passes through the first pipeline 25, flows to the aseptic water side of the low pressure end, passes through the aseptic water sterile T-type diaphragm valve 10 and then enters the first heat exchanger 26 for heat exchange and mixing, and then passes through the third heat exchanger 28 to cool to 50 °C to be supplied to the aseptic filling machine 17 together for use; as the first pipeline 25 gradually supplements the aseptic high-temperature water into the aseptic water circulation system, the flow of the self-supplied aseptic water in the aseptic water circulation system is gradually reduced, and at the same time room-temperature pure water is added to the high-temperature water circulation system.

In the aseptic water circulation system, as the production capacity is gradually reduced, the amount of heat exchange steam used for heating and sterilization will also be reduced, effectively reducing the energy consumption index of the steam, ice water and tower water of the aseptic water circulation system during operation, thus greatly reducing the operation input cost, and as long as the aseptic water circulation system can always ensure the normal operation of the small flow rate, and ensure the aseptic environment is not destroyed, the smaller the self-flow rate is reduced, the lower the overall operating energy consumption; at the same time, the high-temperature water circulating in the high-temperature water circulation system will also be reduced, and pure water at room temperature to the high-temperature water circulation system is added to directly recover heat and exchange heat with the high-temperature material in the material circulation system , and the newly added normal-temperature pure water has a lower temperature than the high-temperature water that is normally backflowed, the heat recovery and cooling capacity on the materials will be better, and the temperature of the materials can be lowered lower, and it can effectively reduce the energy consumption of media such as tower water and ice water at the rear end of material cooling.

The embodiments described above are only for illustrating the technical concepts and features of the present invention, and are intended to make those skilled in the art being able to understand the present invention and thereby implement it, and should not be concluded to limit the protective scope of this invention. Any equivalent variations or modifications of the present invention should be covered by the protective scope of the claims.

## Claims

1. A sterilizing process used for a sterile filling production line, is **characterized in that**, it comprises the following step:
(1) providing a first pipeline (25) between a high-temperature water circulation system and an aseptic water circulation system, and conveying part of high-temperature water that having exchanged heat with a material is to the aseptic water circulation system through the first pipeline (25);
is **characterized in that**, it also comprises the following steps:
(2) supplementing the aseptic water circulation system with pure water, and circulating the pure water in the aseptic water circulation system and heating it to a second sterilization temperature; respectively blocking the communication states between the first pipeline (25) and the high-temperature water circulation system and the aseptic water circulation system, then inletting high-temperature water steam with the second sterilization temperature into the first pipeline (25), until the high-temperature water steam in the first pipeline (25) is kept at the first temperature for a second time period; supplementing the material circulation system with pure water, and supplementing the high-temperature water circulation system with pure water as well, and circulating the pure water in the material circulation system and the high-temperature water circulation system and heating it to the second sterilization temperature; pre-cleaning a material circulation system, the high-temperature water circulation system, the aseptic water circulation system and the first pipeline (25), and after the cleaning is qualified, running the material circulation system, the high-temperature water circulation system and the aseptic water circulation system and communicating the high-temperature water circulation system and the aseptic water circulation system to the first pipeline; when the material circulation system and the aseptic water circulation system are both in the normal production state, the first pipeline (25) is communicated to the high-temperature water circulation system and the aseptic water circulation system; (3) in the high-temperature water circulation system, when the high-temperature water is heated to a first sterilization temperature, sterilizing the high-temperature water for a first time period of heat preservation to obtain aseptic high-temperature water; in the high-temperature water circulation system, before the high-temperature water exchanges heat with steam, it is divided into two paths, the first path directly exchanges heat with the steam, and the second path first exchanges heat with the first path that having exchanged heat with steam, and then flows back into the first path before heat exchange with steam, the first path exchanges heat with the material after exchanging heat with the second path, and a temperature of the first path during heat exchange with the material is a first set temperature; after the first path exchanges heat with the steam, it is heated to the first sterilization temperature, and the temperature is maintained for the first time period; the first set temperature is higher than a temperature of the material exchanging heat with the first path within 3 °C; a material-hot water temperature regulation proportional control valve (7) is provided in the high-temperature water circulation system to regulate the first set temperature;
(4) as the first pipeline (25) gradually supplements the aseptic high-temperature water into the aseptic water circulation system, gradually reducing the flow of the self-supplied aseptic water in the aseptic water circulation system, and at the same time, supplementing the high-temperature water circulation system with room-temperature pure water;
a material-hot water system constant pressure valve (20) and an aseptic hot water supply constant pressure valve (18) are provided in the high-temperature water circulation system, and the material-hot water system constant pressure valve (20) is located upstream of the communication part of the first pipeline (25) and the high-temperature water circulation system, the aseptic hot water supply constant pressure valve (18) is located downstream of the communication part of the first pipeline (25) and the high-temperature water circulation system; an aseptic water cooling system constant pressure valve (24) is provided in the aseptic water circulation system, and the aseptic water cooling system constant pressure valve (24) is located downstream of the communication part of the first pipeline (25) and the aseptic water circulation system; the back pressure of the material-hot water system constant pressure valve (20) is greater than the back pressure of the aseptic hot water supply constant pressure valve (18) which is greater than the back pressure of the aseptic water cooling system constant pressure valve (24) so that the first pipeline (25) can replenish aseptic high-temperature water into the aseptic water circulation system;
the water supply of the aseptic water circulation system is provided by an aseptic water SIP tank (13) or an aseptic water balancing tank (15), and the aseptic water sequentially passes through a first heat exchanger (26), a second heat exchanger (27), a first steam heat exchanger (36), and then flows back through the second heat exchanger (27), the first heat exchanger (26), a third heat exchanger (28), and an aseptic filling machine (17), then returns to the aseptic water SIP tank (13) or the aseptic water balancing tank (15), the first heat exchanger (26) and the second heat exchanger (27) are used to exchange heat between the aseptic water in the shell side and the tube side, and the first steam heat exchanger (36) is used to circulate and heat the aseptic water in the aseptic water circulation system to a second sterilization temperature to obtain the aseptic water, the aseptic water cooled by the third heat exchanger (28) enters the aseptic filling machine (17) for cleaning packaging materials;
the materials of the material circulation system is supplied by a material balancing tank (2), the materials passes through a fourth heat exchanger (29) to heat up and then passes through a degassing homogenizer (5), a fifth heat exchanger (30), and a sixth heat exchanger (31) to heat up and sterilize, the sterilized materials passes through a seventh heat exchanger (32) to heat recovery, then passes through an eighth heat exchanger (33), and a ninth heat exchanger (34) to cool down, and then goes through the aseptic filling machine (17) for filling;
the hot water of the high-temperature water circulation system is supplied by a hot water tank (6), the hot water first passes through the seventh heat exchanger (32) to perform a heat recovery with the high-temperature material of the material circulation system, then the hot water is divided into two paths, the first path directly enters a second steam heat exchanger (37) to heat up and sterilize, and then enters a tenth heat exchanger (35), and the second path enters the tenth heat exchanger (35) to adjust the temperature of the first path, so that the temperature of the high-temperature water output from the tenth heat exchanger (35) and about to enter the sixth heat exchanger (31) is higher than the temperature of the material output from the sixth heat exchanger (31) within 3 °C; the high-temperature water after the heat exchange in the sixth heat exchanger (31) exchanges heat for the low-temperature materials in the material circulation system, and partly returns to the hot water tank (6), and partly enters the first heat exchanger (26) through the first pipeline (25), mixes with the aseptic water in the first heat exchanger (26) and exchanges heat, and then passes through the third heat exchanger (28) to cool down, and then supplies together with the aseptic filing machine for use.

2. The sterilizing process used for a sterile filling production line according to claim 1, is **characterized in that**, in the high-temperature water circulation system, the high-temperature water that having exchanged heat with the material enters the aseptic water circulation system through the first pipeline (25), and then is cooled for use.

3. The sterilizing process used for a sterile filling production line according to claim 1, is **characterized in that**, in step (4), the supplemented room-temperature pure water is mixed with the high-temperature water, and then directly exchanges heat with the material to cool the material.

## Patentansprüche

1. Ein Sterilisationsverfahren, das für eine Produktionslinie zur sterilen Abfüllung verwendet wird, ist **dadurch gekennzeichnet, dass** es den folgenden Schritt beinhaltet:
(1) Bereitstellen einer ersten Pipeline (25) zwischen einem Kreislaufsystem für Hochtemperaturwasser und einem Kreislaufsystem für aseptisches Wasser, und wobei Befördern eines Teils des Hochtemperaturwassers, das Wärme mit einem Material ausgetauscht hat, durch die erste Pipeline (25) zu dem Kreislaufsystem für aseptisches Wasser erfolgt;
ist **dadurch gekennzeichnet, dass** es auch die folgenden Schritte beinhaltet:
(2) Aufstocken des Kreislaufsystems für aseptisches Wasser mit reinem Wasser und Zirkulierenlassen des reinen Wassers in dem Kreislaufsystem für aseptisches Wasser und Erwärmen davon auf eine zweite Sterilisierungstemperatur; jeweils Blockieren der Kommunikationszustände zwischen der ersten Pipeline (25) und dem Kreislaufsystem für Hochtemperaturwasser und dem Kreislaufsystem für aseptisches Wasser, dann Einlassen von Hochtemperaturwasserdampf mit der zweiten Sterilisierungstemperatur in die erste Pipeline (25), bis der Hochtemperaturwasserdampf in der ersten Pipeline (25) für einen zweiten Zeitraum bei der ersten Temperatur gehalten wird; Aufstocken des Materialkreislaufsystems mit reinem Wasser und Aufstocken des Kreislaufsystems für Hochtemperaturwasser ebenfalls mit reinem Wasser und Zirkulierenlassen des reinen Wassers in dem Materialkreislaufsystem und dem Kreislaufsystem mit Hochtemperaturwasser und Erwärmen davon auf die zweite Sterilisierungstemperatur; Vorreinigen eines Materialkreislaufsystems, des Kreislaufsystems für Hochtemperaturwasser, des Kreislaufsystems für aseptisches Wasser und der ersten Pipeline (25), und wenn das Reinigen fertig ist, Laufenlassen des Materialkreislaufsystems, des Kreislaufsystems für Hochtemperaturwasser und des Kreislaufsystems für aseptisches Wasser und Kommunizierenlassen des Kreislaufsystems für Hochtemperaturwasser und des Kreislaufsystems für aseptisches Wasser mit der ersten Pipeline; wenn das Materialkreislaufsystem und das Kreislaufsystem für aseptisches Wasser beide im normalen Produktionszustand vorliegen, wird die erste Pipeline (25) mit dem Kreislaufsystem für Hochtemperaturwasser und dem Kreislaufsystems für aseptisches Wasser in Kommunikation gebracht; (3) in dem Kreislaufsystem für Hochtemperaturwasser, wenn das Hochtemperaturwasser auf eine erste Sterilisierungstemperatur erwärmt ist, Sterilisieren des Hochtemperaturwassers für einen ersten Zeitraum der Wärmekonservierung, um aseptisches Hochtemperaturwasser zu erhalten; wobei, in dem Kreislaufsystem für Hochtemperaturwasser, bevor das Hochtemperaturwasser Wärme mit Dampf austauscht, es auf zwei Pfade aufgeteilt wird, wobei der erste Pfad Wärme direkt mit dem Dampf austauscht und der zweite Pfad zuerst Wärme mit dem ersten Pfad austauscht, der Wärme mit Dampf ausgetauscht hat, und dann vor dem Wärmeaustausch mit Dampf zurück in den ersten Pfad strömt, wobei der erste Pfad nach dem Austauschen von Wärme mit dem zweiten Pfad Wärme mit dem Material austauscht und eine Temperatur des ersten Pfads im Laufe des Wärmeaustauschs mit dem Material eine erste eingestellte Temperatur ist; nachdem der erste Pfad Wärme mit Dampf ausgetauscht hat, wird er auf die erste Sterilisierungstemperatur erwärmt, und die Temperatur wird für den ersten Zeitraum aufrechterhalten; die erste eingestellte Temperatur ist innerhalb von 3 °C höher als eine Temperatur des Materials, das Wärme mit dem ersten Pfad austauscht; ein proportionales Steuerventil (7) zur Regulierung der Material-Heißwassertemperatur ist in dem Kreislaufsystem für Hochtemperaturwasser bereitgestellt, um die erste eingestellte Temperatur zu regulieren;
(4) wenn die erste Pipeline (25) das Kreislaufsystem für aseptisches Wasser graduell mit aseptischem Hochtemperaturwasser aufstockt, graduelles Verringern der Strömung des selbstzugeführten aseptischen Wassers in dem Kreislaufsystem für aseptisches Wasser, und zu der gleichen Zeit Aufstocken des Kreislaufsystems für Hochtemperaturwasser mit reinem Wasser bei Raumtemperatur;
wobei ein Konstantdruckventil (20) des Material-Heißwassersystems und ein Konstantdruckventil (18) zur Versorgung mit aseptischem Heißwasser in dem Kreislaufsystem für Hochtemperaturwasser bereitgestellt sind und das Konstantdruckventil (20) des Material-Heißwassersystems sich stromaufwärts des Kommunikationsteils der ersten Pipeline (25) und des Kreislaufsystems für Hochtemperaturwasser befindet, das Konstantdruckventil (18) zur Versorgung mit aseptischem Heißwasser sich stromabwärts des Kommunikationsteils der ersten Pipeline (25) und des Kreislaufsystems für Hochtemperaturwasser befindet; wobei ein Konstantdruckventil (24) des Kühlsystems für aseptisches Wasser in dem Kreislaufsystem für aseptisches Wasser bereitgestellt ist und das Konstantdruckventil (24) des Kühlsystems für aseptisches Wasser sich stromabwärts des Kommunikationsteils der ersten Pipeline (25) und des Kreislaufsystems für aseptisches Wasser befindet; der Gegendruck des Konstantdruckventils (20) des Material-Heißwassersystems größer ist als der Gegendruck des Konstantdruckventils (18) zur Versorgung mit aseptischem Heißwasser, der größer ist als der Gegendruck des Konstantdruckventils (24) des Kühlsystems für aseptisches Wasser, sodass die erste Pipeline (25) das Kreislaufsystem für aseptisches Wasser wieder mit aseptischem Hochtemperaturwasser auffüllen kann;
wobei die Wasserversorgung des Kreislaufsystems für aseptisches Wasser von einem SIP-Tank (13) für aseptisches Wasser oder einem Ausgleichstank (15) für aseptisches Wasser bereitgestellt wird und das aseptische Wasser sequenziell einen ersten Wärmeaustauscher (26), einen zweiten Wärmeaustauscher (27), einen ersten Dampf-Wärmeaustauscher (36) passiert und dann zurück durch den zweiten Wärmeaustauscher (27), den ersten Wärmeaustauscher (26), einen dritten Wärmeaustauscher (28) und eine aseptische Abfüllungsmaschine (17) strömt, dann zu dem SIP-Tank (13) für aseptisches Wasser oder dem Ausgleichstank (15) für aseptisches Wasser zurückkehrt, wobei der erste Wärmeaustauscher (26) und der zweite Wärmeaustauscher (27) verwendet werden, um Wärme zwischen dem aseptischen Wasser in der Mantelseite und der Rohrseite auszutauschen, und wobei der erste Dampf-Wärmeaustauscher (36) verwendet wird, um das aseptische Wasser in dem Kreislaufsystem für aseptisches Wasser Zirkulieren zu lassen und auf eine zweite Sterilisierungstemperatur zu erwärmen, um das aseptische Wasser zu erhalten, wobei das von dem dritten Wärmeaustauscher (28) gekühlte aseptische Wasser in die aseptische Abfüllungsmaschine (17) zum Reinigen von Verpackungsmaterialien eintritt; wobei die Materialien des Materialkreislaufsystems von einem Materialausgleichstank (2) zugeführt werden, wobei die Materialien einen vierten Wärmeaustauscher (29) passieren, um aufgewärmt zu werden, und dann einen entgasenden Homogenisierer (5), einen fünften Wärmeaustauscher (30) und einen sechsten Wärmeaustauscher (31) passiert, um aufgewärmt und sterilisiert zu werden, wobei die sterilisierten Materialien einen siebten Wärmeaustauscher (32) zur Wärmerückgewinnung passiert, dann einen achten Wärmeaustauscher (33) und einen neunten Wärmeaustauscher (34) passiert, um abzukühlen, und dann durch die aseptische Abfüllungsmaschine (17) zum Befüllen geht;
wobei das Heißwasser des Kreislaufsystems für Hochtemperaturwasser von einem Heißwassertank (6) zugeführt wird, wobei das Heißwasser den siebten Wärmeaustauscher (32) passiert, um eine Wärmerückgewinnung mit dem Hochtemperaturmaterial des Materialkreislaufsystems durchzuführen, wobei das Heißwasser dann auf zwei Pfade aufgeteilt wird, wobei der erste Pfad in einen zweiten Dampf-Wärmeaustauscher (37) eintritt, um aufgewärmt und sterilisiert zu werden, und dann in einen zehnten Wärmeaustauscher (35) eintritt, und wobei der zweite Pfad in den zehnten Wärmeaustauscher (35) eintritt, um die Temperatur des ersten Pfads so anzupassen, dass die Temperatur des von dem zehnten Wärmeaustauscher (35) ausgegebenen und im Begriff befindlichen, in den sechsten Wärmeaustauscher (31) einzutreten, Hochtemperaturwassers innerhalb von 3 °C höher als die Temperatur des von dem sechsten Wärmeaustauscher (31) ausgegebenen Materials ist; wobei das Hochtemperaturwasser nach dem Wärmeaustausch in dem sechsten Wärmeaustauscher (31) Wärme für die Niedertemperaturmaterialien in dem Materialkreislaufsystem austauscht und teilweise zu dem Heißwassertank (6) zurückkehrt und teilweise durch die erste Pipeline (25) in den ersten Wärmeaustauscher (26) eintritt, sich mit dem aseptischen Wasser in dem ersten Wärmeaustauscher (26) mischt und Wärme austauscht und dann den dritten Wärmeaustauscher (28) passiert, um abzukühlen, und dann zusammen mit der aseptischen Abfüllungsmaschine zur Verwendung zugeführt wird.

2. Sterilisationsverfahren gemäß Anspruch 1, das für eine sterile Abfüllungsproduktionslinie verwendet wird, ist **dadurch gekennzeichnet, dass** in dem Kreislaufsystem für Hochtemperaturwasser das Hochtemperaturwasser, das Wärme mit dem Material ausgetauscht hat, durch die erste Pipeline (25) in das Kreislaufsystem für aseptisches Wasser eintritt und dann zur Verwendung gekühlt wird.

3. Sterilisationsverfahren gemäß Anspruch 1, das für eine sterile Abfüllungsproduktionslinie verwendet wird, ist **dadurch gekennzeichnet, dass** in Schritt (4) das aufgestockte reine Wasser bei Raumtemperatur mit dem Hochtemperaturwasser gemischt wird und dann direkt Wärme mit dem Material austauscht, um das Material zu kühlen.

## Revendications

1. Un procédé de stérilisation utilisé pour une ligne de production de remplissage stérile est **caractérisé en ce qu'**il comprend l'étape suivante :
(1) la fourniture d'une première canalisation (25) entre un système de circulation d'eau à haute température et un système de circulation d'eau aseptique, et l'acheminement d'une partie d'eau à haute température qui a échangé de la chaleur avec un matériau vers le système de circulation d'eau aseptique à travers la première canalisation (25) ;
est **caractérisé en ce qu'**il comprend également les étapes suivantes :
(2) l'ajout d'eau pure au système de circulation d'eau aseptique, et la mise en circulation de l'eau pure dans le système de circulation d'eau aseptique et le chauffage de celle-ci à une deuxième température de stérilisation ; le blocage respectif des états de communication entre la première canalisation (25) et le système de circulation d'eau à haute température et le système de circulation d'eau aseptique, puis l'introduction de vapeur d'eau à haute température à la deuxième température de stérilisation jusque dans la première canalisation (25), jusqu'à ce que la vapeur d'eau à haute température dans la première canalisation (25) soit maintenue à la première température pendant une deuxième période de temps ; l'ajout d'eau pure au système de circulation de matériaux, et l'ajout d'eau pure au système de circulation d'eau à haute température également, et la mise en circulation de l'eau pure dans le système de circulation de matériaux et le système de circulation d'eau à haute température et le chauffage de celle-ci jusqu'à la deuxième température de stérilisation ; le pré-nettoyage d'un système de circulation de matériaux, du système de circulation d'eau à haute température, du système de circulation d'eau aseptique et de la première canalisation (25), et une fois le nettoyage validé, la mise en fonctionnement du système de circulation de matériaux, du système de circulation d'eau à haute température et du système de circulation d'eau aseptique et la mise en communication du système de circulation d'eau à haute température et du système de circulation d'eau aseptique avec la première canalisation ; lorsque le système de circulation de matériaux et le système de circulation d'eau aseptique sont tous deux dans l'état de production normal, la première canalisation (25) est mise en communication avec le système de circulation d'eau à haute température et le système de circulation d'eau aseptique ; (3) dans le système de circulation d'eau à haute température, lorsque l'eau à haute température est chauffée jusqu'à une première température de stérilisation, la stérilisation de l'eau à haute température pendant une première période de temps de conservation de chaleur afin d'obtenir de l'eau aseptique à haute température ; dans le système de circulation d'eau à haute température, avant que l'eau à haute température n'échange de la chaleur avec de la vapeur, elle est divisée jusqu'en deux voies, la première voie échange directement de la chaleur avec la vapeur, et la deuxième voie échange d'abord de la chaleur avec la première voie qui a échangé de la chaleur avec la vapeur, puis reflue jusque dans la première voie avant l'échange de chaleur avec de la vapeur, la première voie échange de la chaleur avec le matériau après avoir échangé de la chaleur avec la deuxième voie, et une température de la première voie pendant l'échange de chaleur avec le matériau est une première température définie ; après que la première voie a échangé de la chaleur avec la vapeur, elle est chauffée jusqu'à la première température de stérilisation et la température est maintenue pendant la première période de temps ; la première température définie est supérieure à une température du matériau échangeant de la chaleur avec la première voie dans les limites de 3 °C ; une soupape de commande proportionnelle de régulation de la température du matériau-eau chaude (7) est fournie dans le système de circulation d'eau à haute température afin de réguler la première température définie ;
(4) à mesure que la première canalisation (25) ajoute progressivement l'eau aseptique à haute température jusque dans le système de circulation d'eau aseptique, la réduction progressive du débit de l'eau aseptique auto-alimentée dans le système de circulation d'eau aseptique, et en même temps, l'ajout d'eau pure à température ambiante au système de circulation d'eau à haute température ;
une soupape de pression constante du système matériau-eau chaude (20) et une soupape de pression constante d'alimentation en eau chaude aseptique (18) sont fournies dans le système de circulation d'eau à haute température, et la soupape de pression constante du système matériau-eau chaude (20) est située en amont de la partie de communication de la première canalisation (25) et du système de circulation d'eau à haute température, la soupape de pression constante d'alimentation en eau chaude aseptique (18) est située en aval de la partie de communication de la première canalisation (25) et du système de circulation d'eau à haute température ; une soupape de pression constante du système de refroidissement à eau aseptique (24) est fournie dans le système de circulation d'eau aseptique, et la soupape de pression constante du système de refroidissement à eau aseptique (24) est située en aval de la partie de communication de la première canalisation (25) et du système de circulation d'eau aseptique ; la contre-pression de la soupape de pression constante du système matériau-eau chaude (20) est supérieure à la contre-pression de la soupape de pression constante d'alimentation en eau chaude aseptique (18) qui est supérieure à la contre-pression de la soupape de pression constante du système de refroidissement à eau aseptique (24) de telle sorte que la première canalisation (25) peut réapprovisionner le système de circulation d'eau aseptique en eau aseptique à haute température ;
l'alimentation en eau du système de circulation d'eau aseptique est fournie par un réservoir SIP d'eau aseptique (13) ou un réservoir d'équilibrage d'eau aseptique (15), et l'eau aseptique passe séquentiellement à travers un premier échangeur de chaleur (26), un deuxième échangeur de chaleur (27), un premier échangeur de chaleur à vapeur (36), puis reflue à travers le deuxième échangeur de chaleur (27), le premier échangeur de chaleur (26), un troisième échangeur de chaleur (28) et une machine de remplissage aseptique (17), puis retourne vers le réservoir SIP d'eau aseptique (13) ou le réservoir d'équilibrage d'eau aseptique (15), le premier échangeur de chaleur (26) et le deuxième échangeur de chaleur (27) sont utilisés afin d'échanger de la chaleur entre l'eau aseptique dans le côté enveloppe et le côté tube, et le premier échangeur de chaleur à vapeur (36) est utilisé afin de mettre en circulation et chauffer l'eau aseptique dans le système de circulation d'eau aseptique jusqu'à une deuxième température de stérilisation afin d'obtenir l'eau aseptique, l'eau aseptique refroidie par le troisième échangeur de chaleur (28) entre dans la machine de remplissage aseptique (17) pour nettoyer des matériaux d'emballage ;
les matériaux du système de circulation de matériaux sont fournis par un réservoir d'équilibrage de matériaux (2), les matériaux passent à travers un quatrième échangeur de chaleur (29) afin d'être chauffés, puis passent à travers un homogénéisateur de dégazage (5), un cinquième échangeur de chaleur (30) et un sixième échangeur de chaleur (31) afin d'être chauffés et stérilisés, les matériaux stérilisés passent à travers un septième échangeur de chaleur (32) afin de récupérer la chaleur, puis passent à travers un huitième échangeur de chaleur (33) et un neuvième échangeur de chaleur (34) afin d'être refroidis, puis passent à travers la machine de remplissage aseptique (17) pour être remplis ;
l'eau chaude du système de circulation d'eau à haute température est alimentée par un réservoir d'eau chaude (6), l'eau chaude passe d'abord à travers le septième échangeur de chaleur (32) afin d'effectuer une récupération de chaleur avec le matériau à haute température du système de circulation de matériaux, puis l'eau chaude est divisée jusqu'en deux voies, la première voie entre directement dans un deuxième échangeur de chaleur à vapeur (37) afin d'être chauffée et stérilisée, puis entre dans un dixième échangeur de chaleur (35), et la deuxième voie entre dans le dixième échangeur de chaleur (35) afin d'ajuster la température de la première voie, de telle sorte que la température de l'eau à haute température sortant du dixième échangeur de chaleur (35) et sur le point d'entrer dans le sixième échangeur de chaleur (31) soit supérieure à la température du matériau sortant du sixième échangeur de chaleur (31) dans les limites de 3 °C ; l'eau à haute température après l'échange de chaleur dans le sixième échangeur de chaleur (31) échange de la chaleur pour les matériaux à basse température dans le système de circulation de matériaux, et retourne en partie vers le réservoir d'eau chaude (6), et entre en partie dans le premier échangeur de chaleur (26) à travers la première canalisation (25), se mélange à l'eau aseptique dans le premier échangeur de chaleur (26) et échange de la chaleur, puis passe à travers le troisième échangeur de chaleur (28) afin de se refroidir, puis alimente conjointement avec la machine de remplissage aseptique pour utilisation.

2. Le procédé de stérilisation utilisé pour une ligne de production de remplissage stérile selon la revendication 1 est **caractérisé en ce que**, dans le système de circulation d'eau à haute température, l'eau à haute température qui a échangé de la chaleur avec le matériau entre dans le système de circulation d'eau aseptique à travers la première canalisation (25), puis est refroidie pour utilisation.

3. Le procédé de stérilisation utilisé pour une ligne de production de remplissage stérile selon la revendication 1 est **caractérisé en ce que**, à l'étape (4), l'eau pure à température ambiante ajoutée est mélangée à l'eau à haute température, puis échange directement de la chaleur avec le matériau afin de refroidir le matériau.
